# EUROPEAN PATENT SPECIFICATION

(11) **EP 1 395 600 B1**
(45) Date of publication and mention of the grant of the patent: **07.11.2007**
(21) Application number: 02727809.2
(22) Date of filing: 09.05.2002
(51) Int. Cl.: A61K 51/04, G01N 33/534, B01L 3/02

(54) **PRODUCTS CONTAINING CHARGED BIOMATERIALS AND METHOD FOR THE PREPARATION THEREOF**
GELADENE BIOMATERIALIEN ENTHALTENDE PRODUKTE UND VERFAHREN ZU DEREN HERSTELLUNG
PRODUITS CONTENANT DES BIOMATERIAUX CHARGES, ET PROCEDE D'ELABORATION

(30) Priority: 10.05.2001 HU 0101921 P
(43) Date of publication of application: 10.03.2004
(73) Proprietor: Izotop Intezet Kft., 1121 Budapest (HU)
(72) Inventor: FORSTER, Tibor, H-2151 Fot (HU)
(74) Representative: Machytka-Frank, Daisy
(86) International application number: PCT/HU2002/000039
(87) International publication number: WO 2002/090372

(56) References cited:
- EP-A- 0 513 510
- WO-A-98/37949
- WO-A-02/074840
- DE-A- 3 717 209
- US-A- 4 358 434
- US-A- 5 494 654

## Description

### TECHNICAL FIELD

The invention relates to novel products containing charged biomaterials and to a method for their preparation, thus, for stabilisation, storing, transportation and pre-dispensing of said biomaterials.

In consequence of the enormous development in the area of life sciences, the use of biomaterials has been increased. A number of problems occur during working with biomaterials which are related to the handling of these compounds.

The different materials, chemicals used in the field of life sciences are generally available in bigger quantities. Thus, the users have to calculate and dispense the quantities of different materials needed in one experiment. For dispensing solutions generally pipettors and disposable plastic tips are used in the field of life sciences. The dispensing is a necessary but time-consuming and boring procedure, especially when the same experiments are repeated from time to time. In the case of radioactive materials, in addition, it is a dangerous process because of the radiation and the contamination risk.

The demand for radiolabelled nucleotides which tend to degrade radiolytically, thermally and biologically is especially increased. It is to be ensured that the original quality of these materials is maintained until their use. Safe transportation and storage of radioactive materials, such as radioactive nucleotides, are very important.

In order to increase the sensitivity of the methods, modern molecular biology primarily requires radiolabelled compounds with high specific activity (practically carrier-free). A recent requirement is the elimination of the traditional transportation method of radiolabelled nucleotides, i.e. transportation in frozen state with dry ice storage. Both the high specific activity and the ambient temperature of transportation are factors which increase degradation.

### BACKGROUND ART

There are several solutions for pre-dispensing or dosing of macroscopic amounts of materials. According to US Patent No. 6,343,717, a disposable pipette is used for storage and dispensing of liquid pharmaceutical or cosmetic products which is pre-filled within the body of the pipette.

However, this solution is not suitable in the field of life sciences because of the very small amounts used. No attempt has been found in the literature to solve this problem.

The degradation of radiolabelled organic compounds, especially the degradation of radiolabelled nucleotides, has represented a huge problem for a long time, for both users and manufacturers. Besides the traditionally applied methods of storage at a temperature of - 20°C or - 80°C and of transportation in dry ice, recently storage and transportation without freezing have become popular which is carried out together with the application of additives protecting the compounds from degradation (stabilisers, radical capturers, inhibitors, antioxidants, etc.)

Technical and patent literature contains a number of proposals for the elimination of these disadvantages.

According to US Patent No. 4,390,517, a solution of a radiolabelled compound is stabilised by adding to the solution a compound having an insoluble backbone, to which a quaternary ammonium group or a water soluble primary, secondary or tertiary aliphatic amine has been bound.

In US Patent No. 4,411,881 thiocarbonylated amines are used as stabilisers.

According to US Patent No. 4,793,987, radiolabelled organic compounds are stabilised with derivatives of pyridine carboxylic acid.

According to US Patent No. 5,738,836, organic compounds labelled with a β-emitting radionuclide are stabilised with a compound selected from the group consisting of heteroaryls, aryls and alkylamines, preferably in combination with a neutral dye.

According to US Patents Nos. 5,811,072 and 5,922,301, radiolabelled amino acids and nucleotides are stabilised with a compound selected from tryptophan, para-aminobenzoate, indoleacetate and the azole group, preferably in combination with various dyes.

The self-decomposition of radiolabelled compounds is discussed in details and several compounds as stabilisers are suggested in the Atomic Energy Review, 10:3-66 (1972).

The disadvantage of all methods mentioned above is that the product contains additives which are in fact unnecessary, in some cases detrimental or disturbing. The concentration of the stabilisers is usually larger than the concentration of the radiolabelled material with orders of magnitude. Further disadvantage appears if the radiolabelled material is in solution, since in some applications even water, used as volume increasing material, is disturbing, and the user has to dry the samples. Another disadvantage of radiolabelled materials being in the form of solution proposed by the above mentioned procedures is that the user must divide the samples which represents a danger of contamination.

A further disadvantage of being the radiolabelled compound in the form of a solution is that the shipment and the recovery of the compound by the users require the application of special containers. US Patent No. 5,783,832 proposes a packaging system constituted by three parts. The very internal container is a centrifuge tube which allows collection of the material which gets onto the internal surfaces of the tube during transport. The disadvantage of this method is that the user must perform centrifuging before the use of the material which is, in case of radioactive materials, a dangerous and inconvenient operation, moreover, causes loss of time.

According to US Patent No. 5,922,301, getting the material onto the internal surfaces during transportation is prevented by a splash guard to contain the liquid contents at the bottom of the vial. The disadvantage of the solution proposed by this patent specification is that it minimises the contamination of the inner surfaces but does not prevent it, therefore a part of the material is lost for the users.

The manufacturers search continually the optimal solutions for transportation, storage and stabilisation of radiolabelled nucleotides. Radiolabelled nucleotides are very unstable and become very quickly, as a consequence of radiolytic degradation, inadequate for use in both solid form and in solution.

### DISCLOSURE OF THE INVENTION

The aim of the invention is to find a solution for stabilisation, transportation, storage and pre-dispensing of charged biomaterials, especially radiolabelled nucleotides.

Accordingly, one object of the invention is the conservation of the manufacturing quality of the biomaterials in order to deliver them to the users in the best possible quality. The protection from degradation is especially important in the case of radiolabelled nucleotides.

Another important object of the invention is to find a solution which allows recovery of the biomaterials from the device used for transportation with the best possible efficiency and by the simplest way.

In the case of radiolabelled nucleotides it is also necessary to meet the requirements of the regulations relating to the transportation and storage of radioactive products as dangerous materials.

These aims and tasks should be solved economically, with the lowest possible cost and in the simplest way.

The invention is based on the surprising recognition that the well-known ion exchanging process applied in an appropriate ion exchanger layer is a suitable procedure for transferring charged biomaterials into a reversibly immobilised, solid form providing a device for storage, transportation, stabilisation and pre-dispensing of said charged biomaterials.

Ion exchange is a suitable procedure for reversible immobilisation of ionic or ionisable materials. The electrostatic interaction between the ionic groups of the ion exchanger and those of the charged biomaterials dissolved in an appropriate solution is reserved even after the removal of the solvent. Thus, the charged biomaterials can be transferred from a solution to a special solid form. This solid form provides excellent possibility for transportation, storage and pre-dispensing of said biomaterials. Furthermore, the ionic bond can protect the charged groups from degradation.

In the case of radiolabelled nucleotides it was found that although the radiolabelled nucleotides are very unstable materials, they keep their good quality for a long time, even at ambient temperature, if they are attached to an ion exchanger of special type. Phosphate groups of the nucleotides have significant negative charge, thus, it can be achieved with the application of a suitably chosen ion exchanger and solvent that a given radiolabelled nucleotide will be present in dissolved or to the ion exchanger electrostatically bound form. Using appropriate solvent composition, suppliers can bind the charged biomaterials to the ion exchanger and users can elute them from the ion exchanger. Between these two events the charged biomaterials can be stored and transported in a dry state, bound to the ion exchanger. The product containing the charged biomaterial(s) bound to a solid ion exchanger allows the storage, transportation and use of these materials.

It has been found that all the above-mentioned requirements of storage, transportation, stabilisation and pre-dispensing of charged biomaterials can be fulfilled if the ion exchanger is immobilised on the internal surface of a plastic pipette tip.

Accordingly, the invention provides a product in dried form containing a pre-dispensed quantity of one or more charged biomaterial(s) and optionally an indifferent dye reversibly bound to an ion exchanger immobilised on the internal surface of a plastic pipette tip.

Moreover, the invention provides a kit containing charged biomaterials comprising one or more charged biomaterial(s) in pre-dispensed quantity and optionally an indifferent dye reversibly immobilised to an ion exchanger attached to the internal surface of a plastic pipette tip.

The invention also provides a method for preparing the above mentioned products comprising the following steps:
a) immobilising a pre-dispensed quantity of one or more charged biomaterial(s) on an ion exchanger attached to the internal surface of a plastic pipette tip by treating the ion exchanger with a suitable solution of one or more charged biomaterial(s),
b) optionally immobilising an indifferent dye onto the ion exchanger by treating the ion exchanger with a suitable solution of the dye,
c) removing the solution and
d) drying the immobilised biomaterial(s)/ion exchanger system.

The optional immobilisation of the dye can be performed prior, simultaneously or subsequently to the immobilisation of the biomaterial(s).

Furthermore, the invention provides processes for stabilising and storing charged biomaterials which comprise reversibly immobilising/binding one or more charged biomaterial(s) to an ion exchanger attached to the internal surface of a plastic pipette tip and drying the immobilised biomaterial(s)/ion exchanger system.

In the present application under the term "biomaterial" any material is meant which is involved in the maintenance and metabolic processes of living organisms, including the synthetic analogs and radiolabelled forms thereof, e.g. proteins, enzymes, antibodies, antigens, peptides, amino acids, saccharides, sugars, lipids, fatty acids, drugs, ligands, nucleic acids, oligonucleotides, nucleotides, conjugates or mixtures thereof, etc.

Under the term "charged biomaterial" any biomaterial is meant that have or can have ionic form.

### MODES FOR CARRYING OUT THE INVENTION

Preferably, the accomplishment of the invention is an application of the principles of the batch ion exchanging procedure in a pipette tip.

According to the invention any type of ion exchanger can be used. Depending on the charge of the biomaterial(s) (negative or positive charge) anion or cation exchanger is to be used for immobilisation. The pH dependence of the ionic charge and that of the stability of the biomaterial(s) will determine the type of ion exchanger (weak or strong) to be used. The ion exchange is an equilibrium process and according to the rules of ion exchange, the immobilisation of charged biomaterials onto the ion exchanger is carried out in a solution of low ionic strength and contrary elution of the charged biomaterials from the ion exchanger is carried out in a solution of high ionic strength.

Applying a solution of charged biomaterials at appropriate concentration the pre-dispensing of said biomaterials is possible, that is, as much quantity of charged biomaterials is immobilised in one tip as needed in one experiment. As it is possible to immobilise more pre-dispensed biomaterials in one pipette tip or more tips can be eluted with the same eluent, various reagent kits consisting of charged biomaterials immobilised on pipette tip(s) can be produced.

In a preferred embodiment of the invention, the product according to the invention contains a weak anion exchanger, preferably polyethylenimine or a compound with diethylaminoethyl groups. The use of weak anion exchangers is favourable for attaching nucleotides, since they allow gentler handling than the strong anion exchangers.

For the purposes of the present application the WAX-Tip pipette tips (manufacturer: Institute of Isotopes Ltd., Budapest, Hungary) can be successfully used (this pipette tip is described in details in Hungarian Patent Application No. P 0101145). The internal surface of this pipette tip is coated with immobilised polyethylenimine layer to a height corresponding to a volume of 0.01 ml.

The ion exchanger immobilised on the surface of a plastic pipette tip can be prepared e.g. with the process described in details in Hungarian Patent Application No. P 01 01145. According to this patent application, the process for coating the surface of plastics comprises the following steps:
i) absorbing one or more internal reagent(s) in the plastic to be coated,
ii) contacting one or more external reagent(s) with the surface of said plastic,
iii) forming an immobilised coating by a chemical reaction between the internal reagent(s) and the external reagent(s) both diffusing to the boundary layer of said plastic, preferably in the presence of an additive.

Polyethylenimine, as weak anion exchanger, facilitates the binding of radiolabelled biomaterial(s) to the ion exchanger, and the recovery thereof, since its capacity changes with changing the pH value, furthermore, it contains primary, secondary and tertiary amine groups, which inhibit self-oxidation. As a result of immobilization on the ion exchanger along with the self-oxidation decreasing effect of the matrix of the ion exchanger, the nucleotides immobilised in this way can be stored and transported even at ambient temperature and their stability is the same or even higher than the stability of radiolabelled nucleotides in solutions containing stabilisers or stored in frozen state. At the same time the product proposed by the invention eliminates the disadvantage of stabilisation by freezing or adding stabilisers, thereby avoiding contamination of the radioactive material with an inactive ballast of large quantity.

In a preferred embodiment of the invention the product contains also an indifferent dye.

Applying an indifferent dye with ionic properties similar to those of the charged biomaterial, the dye will indicate the extent of the adsorption/desorption process. That is, the coloured loading solution turns colourless and the colourless layer of the tip becomes coloured by the end of the immobilisation. Contrary, the coloured layer of tip turns colourless and the colourless eluate becomes coloured during the elution.

In a preferred embodiment of the invention the product contains one or more charged biomaterial(s) selected from the group consisting of nucleic acids, oligonucleotides and nucleotides.

Preferably, the product according to the invention contains one or more nucleotide(s) radiolabelled with one or more isotopes selected from the group consisting of H-3, C-14, P-32, P-33, S-35, 1-125.

Preferably the kit according to the invention contains a weak ion exchanger, preferably polyethylenimine or a compound with diethylaminoethyl groups. Preferably the kit contains one or more charged biomaterial(s) selected from the group consisting of nucleic acids, oligonucleotides and nucleotides. More preferably the charged biomaterial(s) is(are) radiolabelled nucleotide(s).

In a preferred embodiment of the method according to invention a product containing radiolabelled nucleotide(s) as biomaterial(s) is prepared and the ion exchanger containing the radiolabelled nucleotide(s) is rinsed with a solution preventing the degradation of the nucleotides.

Optionally, the ion exchanger is pre-treated with a concentrated solution of counter-ion having less affinity to the ion exchanger than the biomatererial(s) to be immobilised. Preferably, the pre-treatment of the ion exchanger also contains the following steps: removal of excess salt by washing with distilled water, equilibrating with the loading buffer, and, in case of postponed use, washing with alcohol and drying.

Storage, transportation and stabilisation in the form of the products according to the invention essentially differs from storage, transportation and stabilisation methods applied up to now (freezing at -20°C temperature or stabilisation with additives in solution). The stability of radiolabelled nucleotides being in the form proposed by the invention, i.e. bound to an ion exchanger, is the same or even higher than their stability in the form produced by the traditional methods. The high stability of the product is probably due to the specifically dispersed form, the ionic bond of phosphate groups of high energy content as well as the interactions between the sugar and base components of the nucleotides and the matrix of the ion exchanger.

Probably another cause of the high stability of the product of the invention is the lack of water. Namely, a significant part of the radioactive radiation of radiolabelled biomaterials prepared in solution is absorbed by the solvent and simultaneously radicals, ions, peroxides and other chemical entities form in great amount which damage the radiolabelled biomaterials.

Preferably, the pipette tip contains the biomaterial(s) in a quantity sufficient for one experiment, only. Thus, the user does not need to dispense the sample which is always accompanied by losses and by the change of the parameters because of the evaporation of the sample. A further advantage of this solution is that the user can simply wash off the biomaterial(s) with a solution of high ionic strength from the pipette tip. For this purpose, for example, the buffer solution of the enzyme reaction related to the application of the biomaterial(s) can also be used. In this way the user is relieved of the problematic dispensing of the biomaterials, especially the radiolabelled materials, and the biomaterials can be added to the system without increasing the volume of the reaction mixture.

A further advantage of the invention, in comparison with the methods applied up to now, is that as a consequence of the immobilising to the ion exchange layer, the biomaterial is in a dry state, without solvent, therefore, its transportation and packaging is simpler and less expensive. According to transport regulations of radioactive materials, radioactivity limit for "excepted" packages 10 times higher for solids then for liquids. In the case of radiolabelled materials elimination of problems arising in the case of cooling with dry ice or of the transportation of liquids (e.g. smearing of the material on the internal surfaces, difficult recovery of the material, getting of the material into the environment in case of accidents, etc.) is not needed.

Due to the application of the product according to the invention the use of the radiolabelled material is significantly simpler, since it is not necessary to wait until the material melts. In addition, there is no contamination danger because of the possible dripping of the material or the turning over of the open container.

Further advantages of the product of the invention over the frozen preparations or the preparations stabilised in a solution are that any unnecessary material, including water, will not get into the user's system. In contrast to this, the radioactive materials prepared by the conventional methods may contain unnecessary, sometimes disturbing components in significant quantities, even up to the magnitude of 10 mM concentration.

A great advantage of the invention is that the biomaterial(s) of the product according to the invention will be purified, since the process of immobilization-eluting in fact represents an ion exchange purification.

The invention is demonstrated with the following examples, without limiting the scope of protection. It should be understood that the foregoing examples merely present a detailed description of certain preferred embodiments. It, therefore, should be apparent to those skilled in the art that various modifications and equivalents can be made without departing from the spirit and scope of the invention.

### Reference example

### Preparation of the ion exchanger immobilised on the surface of a plastic pipette tip

An open vessel of 2 ml is placed into a vessel of 20 ml with a screw cap. A mixture consisting of 5 µl of oxalyl chloride (internal reagent) and 100 µl of trichloroethylene (neutral solvent) are placed into the open vessel of 2 ml. Three pipette tips of 200 µl volume made of polypropylene are placed into the vessel of 20 ml next to the vessel containing the reagent. After closing the external vessel, the system is incubated for overnight. 30 µl of 3% aqueous solution of polyethylenimine as external reagent is sucked into the pipette tips, then the tips are incubated overnight in a vapour cabinet. After removing the solution, the pipette tips are washed with water and alcohol and are dried for 8 hours at 80°C temperature in a vacuum drying oven.

### Example 1

### General procedure for preparing the products of the invention

### Pre-treatment of the ion exchange layer of pipette tips:

The ion exchanger immobilised on the internal surface of a plastic pipette is treated with 2M NaOH solution for 20 minutes, then rinsed with distilled water until neutral. The ion exchanger is treated twice with 2M acetic acid for 20 minutes, then rinsed with distilled water until neutral. Finally the ion exchanger is rinsed with alcohol and dried. The treatment of the ion exchange layer is carried out by sucking the desired solution into the pipette tip and then the ion exchanger is incubated with the solution. By this procedure ion exchangers of chloride or any other forms are transformed into the more favourable acetate form.

### Immobilisation of biomaterial(s)

Low ionic strength solution (1mM or less) of a biomaterial is sucked into the pre-treated pipette tip and incubated for at least 20 minutes. After the removal of the solution the pipette tip is rinsed with distilled water several times and then dried. The sample is ready for storage or transportation.

### Example 2

### Comparison of different ion exchangers

A test solution of the following composition is added to the pre-treated ion exchangers:
Tris-acetate; 1 mM, pH 5,0
Dithiothreitol; 10 mM
Radiolabelled nucleotide; 100 pmole, ~40,000 Bq (T= Total)

Radiolabelled nucleotides used in the experiments 2(a) to 2 (e) are the products of the Institute of Isotopes Ltd., Budapest, Hungary. Radioactivity remaining in the test solution removed (F= Free) is measured after 20 minutes of incubation. After removing the test solution, the ion exchangers are rinsed several times with distilled water and alcohol. After removing the alcohol, the various ion exchangers are dried, closed and stored overnight in a refrigerator. Next day the ion exchanger products are treated with 2M NaCl solution for 5 minutes. After incubation the radioactivity of the salt solution (B= Bound) is determined. The results are given in Table 1.

### Experiment 2(a)

Nucleotide: Adenosine 5' [γ-³⁵S] thiotriphosphate; [γ-³⁵S-ATP]
Ion exchanger: 10x5 mm DEAE-cellulose TLC plate (Macherey-Nagel, France) in a bottle of 1 ml with a screw cap.

### Experiment 2(b)

Nucleotide: Uridine 5' [α-³³P] triphosphate; [α-³³P-UTP]
Ion exchanger: 10x5 mm PEI-cellulose TLC plate (Merck, Germany) in a bottle of 1 ml with a screw cap.

### Experiment 2(c)

Nucleotide: Adenosine 5' [γ-³²P] triphosphate; [γ-³²P-ATP]
Ion exchanger: 5 mg DEAE-Sephadex (Pharmacia, Sweden) closed in a 200 µl ampoule with a cap.

### Experiment 2(d)

Nucleotide: Adenosine 5' [γ-³²P] triphosphate; [γ-³²P-ATP]
Ion exchanger: 5 mg DEAE-Sephacell (Pharmacia, Sweden), closed in a mini chromatography column used for automated oligonucleotide synthesis.

### Experiment 2(e)

Nucleotide: Adenosine 5' [γ-³²P] triphosphate; [γ-³²P-ATP]
Ion exchanger: WAX-Tip pipette tip (Institute of Isotopes Ltd., Budapest, see: Hungarian Patent Application No. P 0101145) containing polyethylenimine layer on its internal surface immobilised by cross-linking.

**Table 1**

| | F (Bq) | B (Bq) | T (Bq) | (T-F)/T | B/(T-F) | B/T |
|---|---|---|---|---|---|---|
| Example No. 2(a) | 3231 | 24121 | 30245 | 89.3% | 89.3% | 79.8% |
| Example No. 2(b) | 5726 | 58656 | 66167 | 91.3% | 97.0% | 88.6% |
| Example No. 2(c) | 3728 | 32257 | 36971 | 89.9% | 97.0% | 87.3% |
| Example No. 2(d) | 1049 | 37673 | 39491 | 97.3% | 98.0% | 95.4% |
| Example No. 2(e) | 1053 | 40577 | 42012 | 97.5% | 99.1% | 96.6% |

The results show that several ion exchangers are suitable to bind radiolabelled nucleotides effectively ([T-F]/T) as well as to recover the nucleotides from the ion exchanger (B/[T-F]). Total efficiency of the whole procedure (B/T) is also good. Each different system is suitable for safe storage and transportation of bound nucleotides.

There are notable differences between the various systems from the aspect that in examples 2(a) to 2(d), especially in example 2(d), several 100 µl of solutions are necessary for adsorption and eluting of nucleotides, while in example 2(e) these can be performed perfectly with only 10 µl of solution. The other difference between the systems derives from the strengths of adhesion of the ion exchange layers. In examples 2(a) and 2(b) the adhesion of the ion exchange layer is not perfect, separation of the parts which flake off is difficult. In example 2(c) a centrifuge is needed for separating the solid and liquid phase. In example 2(d) the problem does not exist if the mesh size of the filter fits to the ion exchanger.

In contrast to the above, in example 2(e) the separation of the liquid phase does not cause any problem, since the ion exchange layer immobilised by cross-linking adheres firmly to the carrier. The greatest advantage of the use of ion exchanger immobilised on the surface of a pipette tip is the user-friendly simplicity of the application while, at the same time, the nucleotide "container" is also the dispensing device.

### Example 3

### Experiments with WAX-Tip pipette tips

The tips are treated as in example 1 and cytidine 5' [α-³²P] triphosphate [α-³²P-CTP] is immobilised on the ion exchange layer as described above. 10 µl of solutions of various pH value, concentration and substances (Tris-HCI, Tris-acetate, NaOH) are added into the pre-treated and dried tips. In order to achieve the equilibrium between the ion exchange layer and the solution, the tips are incubated for an hour. After incubation the test solution is removed and the quantity of the nucleotides remaining in the solution (F), i.e, the radioactivity of the solution is measured. After eluting with 2 M NaCl solution, the quantity of the nucleotides bound by the ion exchange layer (B) is determined by measuring the radioactivity of the eluate. The sum of the quantities of nucleotides remaining in the solution and nucleotides bound by the ion exchange layer is equal in every case - within the error limits - with the total quantity of nucleotides (T). Table 2 shows the ratio of the nucleotides bound by the ion exchange layer (B/T), with the corresponding concentration values.

**Table 2**

| c [mM] | Cl⁻ (pH 7) | Cl⁻ (pH 5) | AcO⁻(pH5) | OH⁻ (pH is varying) |
|---|---|---|---|---|
| 1 | 99.83% | 98.50% | 98.85% | 98.41% |
| 5 | 97.63% | 98.17% | 97.63% | 95.89% |
| 10 | 93.53% | 97.53% | 97.06% | 94.77% |
| 50 | 36.40% | 85.14% | 95.33% | 90.96% |
| 100 | 8.96% | 51.17% | 94.52% | 86.53% |
| 500 | 1.68% | | 16.57% | 27.99% |
| 1000 | 0.90% | | 4.01% | 1.27% |
| 2000 | 0.53% | | 1.31% | 1.01% |

### Example 4

### Binding of nucleotides to WAX-Tip pipette tips

Pipette tips pre-treated as in example 1 are incubated for various periods of time with [α-³³P-CTP] nucleotides dissolved in 1 mM Tris-acetate buffer solution, and the pipette tips containing immobilised nucleotide are incubated for various periods of time with 0.5 M NaCl solution. Table 3 shows the measured values.

**Table 3**

| Adsorption | | Desorption | |
|---|---|---|---|
| T [min] | F/T | T [min] | F/T |
| 0 | 100.0% | 0 | 0% |
| 0.5 | 46.24% | 0.5 | 98.12% |
| 1 | 34.46% | 1 | 97.83% |
| 2 | 22.60% | 3 | 98.74% |
| 5 | 12.49% | 5 | 98.41% |
| 10 | 1.63% | 15 | 98.80% |
| 20 | 0.40% | - | - |
| 30 | 0.29% | - | - |

The results show that the adsorption-desorption process strongly concentration- and time-dependent.

### Example 5

### Eluting radiolabelled nucleotides from WAX-Tip pipette tips

Radiolabelled nucleotides are bound to WAX-Tip pipette tips, as described in example 2. As radiolabelled nucleotides guanosine 5' [γ-³²P] triphosphate [γ-³²P-GTP] is used. For eluting the nucleotides, the following undiluted buffer solutions common in molecular biology are used, in each case in an amount of 10 µl.

### PNK A: T₄ Polynucleotide Kinase 10x forward reaction buffer (MBI Fermentas, Lithuania)

500 mM Tris-HCl, pH 7.6
100 mM MgCl₂
50 mM DDT
1 mM Spermidine
1 mM EDTA

### PNK B: T₄ Polynucleotide Kinase 10x exchange reaction buffer (MBI Fermentas, Lithuania)

500 mM Imidazole-HCl, pH 6.4
180 mM MgCl₂
50 mM DDT
1 mM Spermidine
1 mM EDTA
1 mM ADP

### TdT: Terminal Deoxynucleotidyl Transferase 5x reaction buffer (MBI Fermentas, Lithuania)

1 M Potassium cacodylate, pH 7.2
5 mM CoCl₂
0.5 mM DDT
0.05% Triton X-100

### Klenow: DNA Polymerase Klenow Fragment 10x reaction buffer (MBI Fermentas, Lithuania)

500 mM Tris-HCI, pH 8.0
50 mM MgCl₂
10 mM DDT

### T3/T7: T3/T7 RNA Polymerase 5x transcription buffer (Promega, USA)

200 mM Tris-HCI, pH 7.9
30 mM MgCl₂
50 mM DDT
50 mM NaCl
10 mM Spermidine

### DNA Polymerase: DNA Polymerase I 10x reaction buffer (MBI Fermentas, Lithuania)

500 mM Tris-HCl, pH 7.5
100 mM MgCl₂
10 mM DDT

Radiolabelled nucleotides are eluted from the pipette tips by tenfold "lifting" of the buffers. Table 4 shows the measured values.

**Table 4**

| **Desorption** | |
|---|---|
| Buffer | F/T |
| PNK A | 100.43% |
| PNK B | 99.31% |
| TdT | 97.87% |
| Klenow | 99.07% |
| T3/T7 | 98.80% |
| DNA Polymerase | 98.61% |

The results show that the buffers tested are perfectly suitable to elute the radiolabelled nucleotides. This result may be generalized, since the other reaction buffers used with the radiolabelled nucleotides contain components of similar concentrations.

### Example 6

### Constancy of the quantity of radiolabelled nucleotides bound to WAX-Tip pipette tips

10 µl of the same [γ-³²P-GTP] solution is added into each of seven pre-treated WAX-Tip pipette tips (marked with 1 to 7). After 20 minutes of incubation the solution is removed from the tips and its activity is measured. Results in Table 5 show that there are only very small differences between the quantities of radioactive nucleotides bound by the WAX-Tip pipette tips. The standard deviation is 0.3%, the value of the variation coefficient is also 0.3%.

**Table 5**

| No. | F | B | B/T | B [µCi] |
|---|---|---|---|---|
| 1 | 25984 | 1 160 734 | 97.8% | 31.37 |
| 2 | 27 306 | 1 159 412 | 97.7% | 31.34 |
| 3 | 31 847 | 1 154 871 | 97.3% | 31.21 |
| 4 | 28 061 | 1 158 657 | 97.6% | 31.32 |
| 5 | 27 563 | 1 159 155 | 97.7% | 31.33 |
| 6 | 28613 | 1 158 105 | 97.6% | 31.30 |
| 7 | 36 162 | 1 150 556 | 97.0% | 31.10 |

### Example 7

### Stability of immobilised, radiolabelled nucleotides during storage of various lenghts of time

50 µCi of the α-³³P Deoxycytidine triphosphate [α-³³P-dCTP], 73 µCi of the α-³²P Adenosine triphosphate [α-³²P-ATP] and 31 µCi of the γ-³²P Guanosine triphosphate [γ-³²P-GTP] are immobilised in WAX-Tip pipette tips. In the case of [γ-³²P-GTP], the rinsing with alcohol before storage is substituted by rinsing with 10 mM alcoholic solution of dithiothreitol. The nucleotides immobilised in the pipette tips are stored in closed bags in a refrigerator at a temperature of +4°C. From time to time a pipette tip containing immobilised nucleotides is analyzed quantitatively and qualitatively. Radiolabelled nucleotides are eluted from the tips by PNK buffer solution. The quantity of the dissolved material is measured by liquid scintillation counter, its quality is analysed by thin layer chromatography. Table 6 shows the measured values.

**Table 6**

| **Day** | Recovery [%] | Purity [%] |
|---|---|---|
| | α-³³P-dCTP | |
| 7 | 98.5 | 98.2 |
| 14 | 99.0 | 97.6 |
| 21 | 99.3 | 97.4 |

| | α-³²P-ATP | |
|---|---|---|
| 0 | 98.0 | 98.4 |
| 8 | 98.0 | 98.7 |
| 15 | 97.2 | 98.6 |
| 18 | 97.1 | 98.6 |

| | γ-³²P-GTP | |
|---|---|---|
| 4 | 99.5 | 98.1 |
| 7 | 99.2 | 99.2 |
| 14 | 99.0 | 98.4 |
| 17 | 99.1 | 99.2 |

In the case of recovery, the quantities are corrected with the loss from radioactive decay. It can be established that the radiolabelled nucleotides bound on the ion exchange layer can be recovered - practically - without loss, even after long storage. The quantity of nucleotides bound irreversibly in the WAX-Tip pipette tips is negligible. The stability of nucleotides immobilised on the ion exchange layer is remarkably good.

In contrast to this, nucleotides stored under the same conditions (at +4°C temperature, in refrigerator) but in solution suffer significant degradation. For 15 days, the purity of α-³²P-ATP stored in solution has decreased to 39.3%, while the purity of γ-³²P-GTP has decreased to 43.6%.

In order to test the transportability of the nucleotides, a pipette tip containing radiolabelled α-³²P-ATP and a pipette tip containing radiolabelled γ-³²P-GTP - after 14 days and 15 days of storage, respectively, in refrigerator - have been stored at ambient temperature for three days, thereby simulating the conditions of transportation. The results of analysis (Table 6) show that the storing at ambient temperature has not caused negative effects. It can be established that the radiolabelled nucleotides bound on the ion exchange layer may be transported without cooling.

### Example 8

### Biological applicability of radiolabelled nucleotides stored in WAX-Tip pipette tips

After 11 days of storage in a refrigerator at +4°C temperature, the radiolabelled nucleotide is eluted with 4 µl of 5x Transcription buffer solution from WAX-Tip pipette tip containing 50 µCi α-³²P Cytidine triphosphate [α-³²P-CTP]. Using the further components of the Transcription kit (Promega, USA), radiolabelled RNA is prepared, according to the manufacturer's protocol.

The quantities of both radiolabelled nucleotides incorporated into the RNA and the nucleotide having remained unchanged are measured. The ratio of these two values shows the biological applicability of the radiolabelled nucleotide. The result is 78.1%, which is significantly higher than the acceptance limit of 55% of the incorporation rate.

The result of a control sample - stored in a frozen state at a temperature of -20°C - is 75.2%. These experiments have proved that the nucleotides bound to an ion exchange layer remain biologically applicable, furthermore, any impurities from the ion exchange layer, which would inhibit enzyme reaction, do not dissolve into the buffer solution.

### Example 9

### Product containing DNA and its use

Lambda DNA (48,502 bp) in 10 mM Tris-HCl (pH 7,8), 10 mM NaCl, 1 mM EDTA (Promega, USA) is diluted 50 times with distilled water. A blue coloured mixture of 1 µl 250 mg/l of Patent Blue V (Fluka, Switzerland) solution, 1 µl of diluted DNA solution (10ng) and 8 µl distilled water is sucked into a WAX-TIP pipette tip pre-treated according to Example 1. After 30 minutes the loading solution is blotted to a filter-paper. The colourless spot on the paper and the blue end of the pipette tip indicates that immobilisation occurred. The tip is washed three times with distilled water and then dried.

The immobilised Lambda DNA is eluted from the pipette tip with 10 µl 10x PCR buffer. The buffer is added into a micro-centrifuge tube of 200 µl, then the buffer is picked up and ejected several times. During this the solution turns blue and the pipette turns colourless. Finally, as a washing, 72 µl of distilled water is sucked into the tip and it is added to the eluted DNA. The PCR reaction mixture (MBI Fermentas, Lithuania) is completed by adding the following component to the mixture:
6 µl 25 mM MgCl₂, 2 µl 10 mM dATP, 2 µl 10 mM dCTP, 2 µl 10 mM dGTP, 2 µl 10 mM dTTP, 1 µl 10µM Lambda 1 primer (CTA CCA TAT CTC CTA TGA TGA GCA ACG), 1 µl 10 µM Lambda 2 primer (GCC TTT GCC TCG CTA TAC ATT TC) and finally 2.5 U Taq DNA Polymerase. Parameters of temperature cycling are the following:
   Initial denaturation: 5 min at 95 °C; Denaturation: 1 min at 95 °C; Primer annealing: 1 min at 55 °C; Extending: 0.5 min at 72 °C; Final extending: 7 min at 72 °C. Number of cycles is 30.

The reaction mixture is analysed by agarose gel electrophoresis. After staining with ethidium bromide, a 540 bp PCR product is visualised showing that the PCR reaction can take place with the eluted DNA template. The same procedure is carried out with an immobilised DNA stored at +4 °C in refrigerator with the very same result.

The results show that DNA bound to an ion exchange layer has the same biological activity as that of the solution form. The immobilised DNA remains biologically applicable even after long time of storage. Application of a dye is suitable for monitoring the adsorption/desorption process. Applying the pre-dispensed form a dispensing step is substituted with a washing step increasing the accuracy of the dosing.

### Example 10

### Product containing oligonucleotides and its use

Immobilisation of two different oligonucleotides is carried out as described in Example 9 with the following loading solution: 1 µl 10µM Lambda 1 primer, 1 µl 10µM Lambda 2 primer, 1 µl 250 mg/l of Patent Blue V solution and 6 µl 1mM Tris-EDTA buffer (pH 7.0).

Immobilisation of radiolabelled nucleotides is carried out as described in Example 9 with the following loading solution: 5 µl (50µCi) α-³²P-dCTP in 0.5 mM Tris-EDTA buffer (pH 7.0), 1 µl 500 mg/l of Sulforhodamine B (Fluka, Switzerland) solution and 4 µl of distilled water.

The complete PCR reaction mixture (10 µl 10x PCR buffer, 6 µl 25 mM MgCl₂, 2 µl 10 mM dATP, 0.5 µl 10 mM dCTP, 2 µl 10 mM dGTP, 2 µl 10 mM dTTP, 1 µl Lambda DNA (10 ng), 76 µl of distilled water and 2.5 U Taq DNA Polymerase) is used for elution of primers at first and then α-³²P-dCTP. The colour of the solution turns at first blue and then lilac indicating the elution of the different compounds. The thermal cycling and the analysis are the same as described in Example 10 supplemented with an autoradiogram image of the agarose gel. Both the ethidium bromide staining and the autoradiogram show the resulting 540 bp PCR amplicon.

The results show that several materials can be immobilised in one pipette tip and several pipette tips can be applied in one experiment. Colour coding of different components helps users in correct preparation of a reaction mixture.

## Claims

1. A product containing at least one charged biomaterial reversibly immobilised to an ion exchanger attached to the internal surface of a plastic pipette tip,
**characterised in that**
the product is in dried form and it contains a pre-dispensed quantity of the at least one biomaterial immobilised to the ion-exchanger.

2. The product according to claim 1, wherein the ion exchanger is a weak anion exchanger.

3. The product according to claim 2, wherein the weak ion exchanger is polyethylenimine or a compound with diethylaminoethyl groups.

4. The product according to any of claims 1 to 3, wherein the at least one charged biomaterial is selected from the group consisting of nucleic acids, oligonucleotides and nucleotides.

5. The product according to any of claims 1 to 4, wherein the at least one charged biomaterial is radiolabelled nucleotide.

6. The product according to claim 5, wherein the nucleotide is radiolabelled with one or more isotopes selected from H-3, C-14, P-32, P-33, S-35,1-125.

7. The product according to any of claims 1 to 6, which further contains an indifferent dye.

8. A kit containing at least one product according to claim 1.

9. The kit according to claim 8, wherein the ion exchanger is a weak anion exchanger.

10. The kit according to claim 9, wherein the weak ion exchanger is polyethylenimine or a compound with diethylaminoethyl groups.

11. The kit according to any of claims 8 to 10, wherein the at least one charged biomaterial is selected from the group consisting of nucleic acids, oligonucleotides and nucleotides.

12. The kit according to any of claims 8 to 11, wherein the at least one charged biomaterial is radiolabelled nucleotide.

13. A method for the preparation of the products according to claim 1, comprising the following steps:
a) immobilising a pre-dispensed quantity of at least one charged biomaterial onto an ion exchanger attached to the internal surface of a plastic pipette tip by treating the ion exchanger with a suitable solution of the at least one charged biomaterial,
b) removing the solution and
c) drying the immobilised at least one biomaterial/ion exchanger system.

14. A method for the preparation of the products according to claim 7, comprising the following steps:
a) immobilising a pre-dispensed quantity of at least one charged biomaterial onto an ion exchanger attached to the internal surface of a plastic pipette tip by treating the ion exchanger with a suitable solution of the at least one charged biomaterial,
b) immobilising an indifferent dye onto the ion exchanger by treating the ion exchanger with a suitable solution of the dye,
c) removing the solution and
d) drying the immobilised at least one biomaterial/ion exchanger system.

15. The method according to claim 14, wherein the immobilisation of the dye is performed prior, simultaneously or subsequently to the immobilisation of the at least one biomaterial.

16. The method according to any of claims 13 to 15, wherein the ion exchanger is a weak anion exchanger.

17. The method according to claim 16, wherein the weak anion exchanger is polyethylenimine or a compound with diethylaminoethyl groups.

18. The method according to any of claims 13 to 17, wherein the at least one charged biomaterial is selected from the group consisting of nucleic acids, oligonucleotides and nucleotides.

19. The method according to any of claims 13 to 18, wherein the at least one charged biomaterial is radiolabelled nucleotide.

20. The method according to claim 19, wherein the ion exchanger containing the at least one radiolabelled nucleotide is rinsed with a solution which prevents the degradation of the at least one radiolabelled nucleotide.

21. Process for stabilising charged biomaterials, comprising immobilising in a reversible form at least one charged biomaterial on an ion exchanger attached to the internal surface of a plastic pipette tip and drying the immobilised at least one biomaterial/ion exchanger system.

22. Process for storing and transporting charged biomaterials, comprising immobilising in a reversible form at least one charged biomaterial on an ion exchanger attached to the internal surface of a plastic pipette tip and drying the at least one immobilised biomaterial/ion exchanger system.

## Patentansprüche

1. Produkt, enthaltend mindestens ein geladenes Biomaterial, das reversibel auf einem Ionenaustauscher immobilisiert ist, der an der inneren Oberfläche einer
Kunststoffpipettenspitze angebracht ist,
**dadurch gekennzeichnet, dass**
das Produkt in getrockneter Form vorliegt und eine vordispergierte Menge des mindestens einen auf dem Ionenaustauscher immobiliesierten Biomaterials enthält.

2. Produkt gemäß Anspruch 1, wobei der Ionenaustauscher ein schwacher Anionenaustauscher ist.

3. Produkt gemäß Anspruch 2, wobei der schwache Ionenaustauscher Polyethylenimin oder eine Verbindung mit Diethylaminoethylgruppen ist.

4. Produkt gemäß einem der Ansprüche 1 bis 3, wobei das mindestens eine geladene Biomaterial aus Nukleinsäuren, Oligonukleotiden und Nukleotiden ausgewählt ist.

5. Produkt gemäß einem der Ansprüche 1 bis 4, wobei das mindestens eine geladene Biomaterial ein radioaktiv gekennzeichnetes Nukleotid ist.

6. Produkt gemäß Anspruch 5, wobei das Nukleotid mit einem oder mehreren Isotopen radioaktiv **gekennzeichnet** ist, ausgewählt aus H-3, C-14, P-32, P-33, S-35, I-125.

7. Produkt gemäß einem der Ansprüche 1 bis 6, welches weiterhin einen reaktionslosen Farbstoff enthält.

8. Kit, enthaltend mindestens ein Produkt gemäß Anspruch 1.

9. Kit gemäß Anspruch 8, wobei der Ionenaustauscher ein schwacher Anionenaustauscher ist.

10. Kit gemäß Anspruch 9, wobei der schwache Ionenaustauscher Polyethylenimin oder eine Verbindung mit Diethylaminoethylgruppen ist.

11. Kit gemäß einem der Ansprüche 8 bis 10, wobei das mindestens eine geladene Biomaterial aus Nukleinsäuren, Oligonukleotiden und Nukleotiden ausgewählt ist.

12. Kit gemäß einem der Ansprüche 8 bis 11, wobei das mindestens eine geladene Biomaterial ein radioaktiv gekennzeichnetes Nukleotid ist.

13. Verfahren zu Herstellung der Produkte gemäß Anspruch 1, umfassend die folgenden Schritte:
a) Immobilisieren einer vordispergierten Menge mindestens eines geladenen Biomaterials auf einen Ionenaustauscher, der an der inneren Oberfläche einer Kunststoffpipettenspitze angebracht ist, durch Behandeln des Ionenaustauschers mit einer geeigneten Lösung mindestens eines geladenen Biomaterials,
b) Entfernen der Lösung und
c) Trocknen des immobilisierten mindestens einen Biomaterials/Ionenaustauschersystems.

14. Verfahren zur Herstellung der Produkte gemäß Anspruch 7, umfassend die folgenden Schritte:
a) Immobilisieren einer vordispergierten Menge mindestens eines geladenen Biomaterials auf einen Ionenaustauscher, der an die Innere Oberfläche einer Kunststoffpipettenspitze angebracht ist, durch Behandeln des Ionenaustauschers mit einer geeigneten Lösung mindestens eines geladenen Biomaterials,
b) Immobilisieren eines reaktionslosen Farbstoffs auf den Ionenaustauscher durch Behandeln des Ionenaustauschers mit einer geeigneten Lösung des Farbstoffs,
c) Entfernen der Lösung und
d) Trocknen des immobilisierten mindestens einen Biomaterials/Ionenaustauschersystems.

15. Verfahren gemäß Anspruch 14, wobei das Immobilisieren des Farbstoffs vor, gleichzeitig mit oder nachfolgend zu der Immobilisierung des mindestens einen Biomaterials durchgeführt wird.

16. Verfahren gemäß einem der Ansprüche 13 bis 15, wobei der Ionenaustauscher ein schwacher Anionenaustauscher ist.

17. Verfahren gemäß Anspruch 16, wobei der schwache Anionenaustauscher Polyethylenimin oder eine Verbindung mit Diethylaminoethylgruppen ist.

18. Verfahren gemäß einem der Ansprüche 13 bis 17, wobei das mindestens eine geladene Biomaterial aus Nukleinsäuren, Oligonukleotiden und Nukleotiden.

19. Verfahren gemäß einem der Ansprüche 13 bis 18, wobei das mindestens eine geladene Biomaterial ein radioaktiv gekennzeichnetes Nukleotid ist.

20. Verfahren gemäß Anspruch 19, wobei der Ionenaustauscher, der das mindestens eine radioaktiv **gekennzeichnet**e Nukleotid enthält, mit einer Lösung gespült wird, die die Zersetzung des mindestens einem radioaktivgekennzeichneten Nukleodis verhindert.

21. Verfahren zum Stabilisieren von geladenen Biomaterialien, umfassend Immobilisieren mindestens eines geladenen Biomaterials in einer reversiblen Form auf einem Ionenaustauscher, der an der inneren Oberfläche einer Kunststoffpipettenspitze angebracht ist, und Trocknen des immobilisierten mindestens einen Biomaterials/lonenaustauschersystems.

22. Verfahren zum Speichern und Transportieren von geladenen Biomaterialien, umfassend Immobilisieren mindestens eines geladenen Biomaterials in einer reversiblen Form auf einem Ionenaustauscher, der an der inneren Oberfläche einer Kunststoffpipettenspitze angebracht ist, und Trocknen des mindestens einen immobilisierten Biomaterials/Ionenaustauschersystems.

## Revendications

1. Produit contenant au moins un biomatériau chargé, immobilisé de manière réversible sur un échangeur d'ions fixé sur la face interne d'une pointe de pipette en plastique,
**caractérisé en ce que**
le produit est sous forme séchée et contient une quantité pré-distribué d'au moins un biomatériau immobilisé sur l'échangeur d'ions.

2. Produit selon la revendication 1, dans lequel l'échangeur d'ions est un échangeur d'anions faible.

3. Produit selon la revendication 2, dans lequel l'échangeur d'ions faible est une polyéthylèneimine ou un composé avec des groupements diéthylaminoéthyle.

4. Produit selon l'une quelconque des revendications 1 à 3, dans lequel le au moins un biomatériau chargé est choisi dans le groupe constitué des acides nucléiques, des oligonucléotides et des nucléotides.

5. Produit selon l'une quelconque des revendications 1 à 4, dans lequel le au moins un biomatériau chargé est un nucléotide radiomarqué.

6. Produit selon la revendication 5, dans lequel le nucléotide est radiomarqué par un ou plusieurs isotopes choisis parmi H-3, C-14, P-32, P-33, S-35, I-125.

7. Produit selon l'une quelconque des revendications 1 à 6, qui contient en outre un colorant quelconque.

8. Trousse contenant au moins un produit selon la revendication 1.

9. Trousse selon la revendication 8, dans laquelle l'échangeur d'ions est un échangeur d'anions faible.

10. Trousse selon la revendication 9, dans laquelle l'échangeur d'ions faible est une polyéthylènemine ou un composé avec des groupements diéthylaminoéthyle.

11. Trousse selon l'une quelconque des revendications 8 à 10, dans laquelle le au moins un biomatériau chargé est choisi dans le groupe constitué des acides nucléiques, des oligonucléotides et des nucléotides.

12. Trousse selon l'une quelconque des revendications 8 à 11, dans laquelle le au moins un biomatériau chargé est un nucléotide radiomarqué.

13. Procédé pour la préparation des produits selon la revendication 1, comprenant les étapes suivantes :
a) l'immobilisation d'une quantité pré-distribuée d'au moins un biomatériau chargé sur un échangeur d'ions fixé à la surface interne d'une pointe de pipette en plastique en traitant l'échangeur d'ions avec une solution appropriée d'au moins un biomatériau chargé,
b) le retrait de la solution et
c) le séchage du système immobilisé du au moins un biomatériau et de l'échangeur d'ions.

14. Procédé pour la préparation des produits selon la revendication 7, comprenant les étapes suivantes :
a) l'immobilisation d'une quantité pré-distribuée d'au moins un biomatériau chargé sur un échangeur d'ions fixé à la surface interne d'une pointe de pipette en plastique en traitant l'échangeur d'ions avec une solution appropriée d'au moins un biomatériau chargé,
b) l'immobilisation d'un colorant quelconque sur l'échangeur d'ions en traitant l'échangeur d'ions avec une solution appropriée du colorant,
c) le retrait de la solution et
c) le séchage du système immobilisé du au moins un biomatériau et de l'échangeur d'ions

15. Procédé selon la revendication 14; dans lequel l'immobilisation du colorant est effectuée avant, pendant ou après l'immobilisation du au moins un biomatériau.

16. Procédé selon l'une quelconque des revendications 13 à 15, dans lequel l'échangeur d'ions est un échangeur d'anions faible.

17. Procédé selon la revendication 16, dans lequel l'échangeur d'anions faible est une polyéthylèneimine ou un composé avec des groupements diéthylaminoéthyle.

18. Procédé selon l'une quelconque des revendications 13 à 17, dans lequel le au moins un biomatériau chargé est choisi dans le groupe constitué des acides nucléiques, des oligonucléotides et des nucléotides.

19. Procédé selon l'une quelconque des revendications 13 à 18, dans lequel le au moins un biomatériau chargé est un nucléotide radiomarqué.

20. Procédé selon la revendication 19, dans lequel l'échangeur d'ions contenant le au moins un nucléotide radiomarqué est rincé avec une solution qui empêche la dégradation du au moins un nucléotide radiomarqué.

21. Procédé pour stabiliser des biomatériaux chargés, comprenant l'immobilisation sous forme réversible d'au moins un biomatériau chargé sur un échangeur d'ions fixé sur la surface interne d'une pointe de pipette en plastique et le séchage du système immobilisé du au moins un biomatériau et de l'échangeur d'ions.

22. Procédé pour stocker et transporter des biomatériaux chargés, comprenant l'immobilisation sous forme réversible d'au moins un biomatériau chargé sur un échangeur d'ions fixé à la surface interne d'une pointe de pipette en plastique et le séchage du système immobilisé du au moins un biomatériau et de l'échangeur d'ions.
